Europäisches Patentamt

European Patent Office

Office européen des brevets

(19)

(11) Numéro de publication: **0 271 747 B1**

(12) **FASCICULE DE BREVET EUROPEEN**

(45) Date de publication de fascicule du brevet: **10.08.94**

(51) Int. Cl.⁵: **C11C 1/00**, C07C 57/12, C07C 51/42, A61K 31/19, A61K 7/48

(21) Numéro de dépôt: **87117362.1**

(22) Date de dépôt: **25.11.87**

Le dossier contient des informations techniques présentées postérieurement au dépôt de la demande et ne figurant pas dans le présent fascicule.

(54) **Procédé de fractionnement en continu d'un mélange d'acides gras.**

(30) Priorité: **17.12.86 CH 5027/86**

(43) Date de publication de la demande:
**22.06.88 Bulletin 88/25**

(45) Mention de la délivrance du brevet:
**10.08.94 Bulletin 94/32**

(84) Etats contractants désignés:
**AT BE DE ES FR GB GR IT LU NL SE**

(56) Documents cités:
**EP-A- 0 178 442**
**US-A- 2 662 879**
**US-A- 4 140 620**

**THE JOURNAL OF THE AMERICAN OIL CHE-MISTS' SOCIETY, vol. 31, janvier 1954, pp. 16-20; A.M. ABU-NASR et al.: "Highly unsatu-rated fatty acids. II. Fractionation by urea inclusion compounds"**

**Fatty acids, D. SWERN, 2nd. edition, K.S. MARKLEY editor, Interscience Publishers, pp.2309-2358 (1964)**

**Fraktionierung mehrfach ungesättigter Fett-säuren aus verschiedenen natürlichen Rohstoffen, H. TRAITLER et al., Fet-te/Seifen/Anstrichmittel, Nr.10 pp.378-382 (1986)**

(73) Titulaire: **SOCIETE DES PRODUITS NESTLE S.A.**
**Case postale 353**
**CH-1800 Vevey(CH)**

(72) Inventeur: **Traitler, Helmut**
**Route de Saint-Légier 10A**
**CH-1800 Vevey(CH)**
Inventeur: **Wille, Hans-Juergen**
**Rue du Port 22**
**CH-1815 Clarens(CH)**

Rank Xerox (UK) Business Services
(3.10/3.09/3.3.3)

**Description**

L'invention se rapporte au fractionnement d'un mélange d'acides gras en vue d'obtenir une fraction enrichie en acides gras polyinsaturés biologiquement actifs.

On sait séparer les acides gras de différents degrés de saturation à partir d'un mélange par formation d'un complexe d'inclusion avec l'urée en présence d'un solvant et séparation de la fraction liquide enrichie d'avec le complexe. Par exemple, le brevet français No. 1.603.383 correspondant au brevet britannique No. 1.240.513 concerne l'enrichissement en acide gamma-linolénique (tri-insaturé) d'un mélange d'acides gras provenant de l'huile d'onagre (Oenothera) qui contient en outre des acides gras de degré d'insaturation moindre.

On sait même enrichir sélectivement en acides gras polyinsaturés dont la première double liaison est en position Δ 6 (par exemple l'acide gamma-linolénique) un mélange d'acides gras contenant en outre des acides gras polyinsaturés dont la première double liaison est en position Δ 9 (par exemple l'acide alpha-linolénique) par la méthode de complexation à l'urée appliquée dans des conditions spécifiques ce qui implique une séparation des isomères de position (voir par exemple la demande de brevet européen publiée No. 178.442). Bien que ce procédé, qui est discontinu, conduise à un enrichissement satisfaisant du point de vue de la sélectivité et du rendement au laboratoire ou au pilote, il est difficile à mettre en oeuvre à l'échelle industrielle, par exemple par charges de plusieurs t: la formation du complexe d'inclusion en cuve s'effectue très lentement. Elle ne s'opère correctement, c'est à dire avec une sélectivité acceptable que sous un brassage aussi lent que possible. De ce fait, les échanges thermiques sont médiocres. Or, un réglage fin de la température réactionnelle est nécessaire pour ne pas produire la redissolution et/ou le vieillissement des cristaux d'urée qui ne sont alors plus capables d'inclure les acides gras et provoquer ainsi une diminution de la sélectivité de l'enrichissement.

L'art antérieur, par exemple dans "Fatty acids, their chemistry, properties, production and uses", D.Swern, pp. 2310-2358, donnait comme nécessaire pour la formation de complexes d'inclusion de l'urée une durée supérieure ou égale à 1 h.

La présente invention se propose d'éliminer les inconvénients ci-dessus.

Elle concerne un procédé de fractionnement en continu d'un mélange d'acides gras ou des dérivés d'acides gras polyinsaturés, dans lequel on fait réagir le mélange avec l'urée en tant qu'agent complexant en solution dans un milieu réactionnel, dans les conditions permettant la formation dudit complexe, à 60-65°C, et l'on forme le dit complexe insoluble en refroidissant le milieu réactionnel, caractérisé par le fait que l'on refroidit le milieu réactionnel dans un à cinq échangeurs à surface râclée placés en série de sorte que la température de la charge a'la sortie du dervier échangeur est de -5 à + 15°C, que la durée de résidence totale de la charge dans les échangeurs est de quelques secondes à quelques minutes, que l'on sépare le complexe d'inclusion formé et que l'on recueille une fraction enrichie en acides gras polyinsaturés dans la phase liquide.

Comme matière première, on peut utiliser un mélange d'acides gras provenant d'une matière grasse d'origine végétale ou animale riche en acides gras polyinsaturés. Parmi les matières grasses mises en oeuvre, on peut citer les huiles riches en acide linoléique (di-insaturé), par exemple l'huile de carthame, de tournesol, de pépins de raisin, de maïs, de soja; les huiles riches en acides gras de degré d'insaturation plus élevé, par exemple l'huile de graines de lin, de consoude (Symphytum officinale), d'onagre (Oenothera), de quandong (Santalum acuminatum), de bourrache (Borago officinalis) ou de pépins de fruits du genre Ribes, par exemple de cassis (Ribes nigrum).

Parmi les matières grasses animales envisagées, on peut citer les huiles de poissons, de crustacés, de céphalopodes et les lipides provenant d'organes ou de fluides corporels de mammifères. Les acides gras peuvent être obtenus à partir des lipides précédents par exemple par hydrolyse à haute température sous forte pression ou, de préférence, par saponification. La saponification peut être réalisée à partir des graines ou des pépins séchés et broyés, à partir des graines ou des pépins séchés mis sous forme de flocons, boulettes ou granules ou encore à partir de l'huile extraite de ces graines ou pépins. Elle peut intervenir sur les lipides d'origine animale sous forme liquide. Ceux-ci peuvent contenir une proportion non négligeable d'insaponifiables qu'il est alors avantageux de séparer.

Les dérivés d'acides gras visés sont de préférence les esters, par exemple les esters méthyliques obtenus par réaction des triglycérides avec le méthylate de sodium. On préfère utiliser les acides gras plutôt que leurs esters car le rendement d'enrichissement est meilleur.

La saponification peut s'effectuer de manière classique en traitant la matière première par une base forte concentrée, par exemple l'hydroxyde de sodium, de préférence en milieu aqueux ou hydro-alcoolique à chaud. Ce milieu contient avantageusement un agent séquestrant des ions métalliques, par exemple l'éthylènediaminetétraacétate disodique. On peut séparer ensuite les insaponifiables par un solvant, par exemple l'hexane et on acidifie la phase aqueuse, par exemple par l'acide chlorhydrique en solution aqueuse concentrée.

Après la saponification, le mélange obtenu peut être protégé contre l'oxydation par l'addition d'un antioxydant, par exemple 100 à 600 ppm (parties par million) de gallate de propyle ou, de préférence, 200 à 400 ppm de palmitate d'ascorbyle.

On peut également utiliser comme produit de départ les savons obtenus lors de la neutralisation de l'huile brute au cours de son raffinage.

Le fractionnement consiste à opérer dans des conditions permettant la formation d'un complexe des acides gras avec l'agent complexant, dans un milieu réactionnel tel que l'agent complexant soit soluble mais pas les complexes d'inclusion formés. Comme agent complexant on utilise l'urée. Le milieu réactionnel peut être constitué d'un bon solvant de l'urée, par exemple un alcanol inférieur, c'est à dire de 1 à 4 atomes de carbone, de préférence le méthanol, l'éthanol, l'isopropanol ou un mélange de ces alcanols avec l'eau. Le méthanol est particulièrement approprié, de même qu'une solution contenant, en poids, envion 85% de méthanol pour environ 15% d'eau.

Dans une cuve munie de moyens d'agitation et de thermostatisation, par exemple par circulation d'eau dans un double manteau, on prépare la solution de préférence saturée par exemple d'urée dans le méthanol et on ajoute les acides gras à 60-65°C sous agitation vigoureuse jusqu'à ce que l'on obtienne une solution limpide. On utilise un rapport pondéral urée: solvant de 1:1,5 à 1:3 et un rapport pondéral acides gras:urée de 1:3 à 1:6 et de préférence de 1:3 à 1:4.

On pompe alors la solution à travers 1 à 5 échangeurs de chaleur à surface raclée placés en série. En variante, on peut remplacer par exemple l'échangeur à surface raclée en bout de ligne par une conduite d'attente munie de moyens de refroidissement.

C'est dans cette ligne que s'effectue la précipitation du complexe insoluble. La solution entrante peut être à 20-65°C, à la température de la dissolution, par exemple à 60-65°C ou encore avoir été refroidie, par exemple à 20-40°C. Les échangeurs de chaleur sont refroidis par un liquide frigorigène en circulation rapide, par exemple l'eau glacée, l'éthanol, le fréon, l'ammoniac dont la température est de - 25 à + 5°C et de préférence de - 16 à + 2°C. Le débit de matière dépend de la capacité de refroidissement des échangeurs de chaleur et du fluide frigorigène utilisé. La vitesse de rotation des éléments râcleurs peut aller jusqu'à 3000 t/min et est de préférence 500 à 1000 t/min. Ainsi le temps de résidence total dans la ligne est de quelques s. à quelques mins. La température finale de la dispersion à la sortie du dernier échangeur joue un rôle déterminant dans la séparation des phases solide et liquide. Selon la nature des acides gras à fractionner elle se situe entre - 5 et 15°C.

Après le traitement dans les échangeurs de chaleur à surface raclée, on dirige avantageusement la dispersion vers une cuve refroidie, par exemple à environ 2°C, puis on sépare la phase solide de la phase liquide par filtration, de préférence sous vide léger ou par centrifugation.

Après élimination de l'alcanol, par exemple par distillation sous vide, on traite la phase liquide par un acide, par exemple l'acide chlorhydrique concentré, de manière à acidifier jusqu'à un pH d'environ 1 pour faire passer en solution aqueuse l'urée n'ayant pas réagi et on ajoute un solvant, par exemple l'hexane pour extraire les acides gras, par exemple par décantation, récupération de la phase organique et élimination du solvant, par exemple par distillation sous vide. On peut recueillir par exemple le méthanol, puis l'hexane qui peuvent être réutilisés.

En variante, on peut se passer de l'une ou des deux étapes d'acidification et d'extraction par un solvant.

La phase solide contient encore des acides gras que l'on peut désirer récupérer. Pour ce faire, on peut ajouter au complexe de l'eau, de préférence dans un rapport pondéral complexe:eau d'environ 1:2, puis chauffer de manière à libérer les acides gras et extraire ensuite ceux-ci de la phase aqueuse, par exemple par l'hexane. On peut éliminer l'hexane de cette deuxième phase organique et joindre les acides gras récupérés aux acides gras de départ devant être fractionnés, ce qui permet d'augmenter le rendement en conséquence.

Quant à l'urée, elle peut être utilisée par exemple comme engrais car elle n'est plus suffisamment active.

Les fractions d'acides gras brutes obtenues peuvent être de préférence décolorées, par exemple par traitement avec 0,5 à 5% en poids de terres décolorantes, de préférence à une température d'environ 80°C. Les fractions décolorées ou non peuvent être ensuite de préférence purifiées, par exemple par distillation à 150-210°C, sous un vide de 13 à 133 Pa (0,1 à 1 mm Hg) dans un appareil à contre courant.

Les fractions d'acides gras, le cas échéant décolorées et/ou purifiées, peuvent être stabilisées en les protégeant de i'oxydation, par exemple par addition d'environ 200 ppm (parties par million) en poids de palmitate d'ascorbyle.

Les fractions d'acides gras obtenues selon l'invention peuvent être utilisées dans les applications de ces acides telles quelles ou sous forme d'huile obtenue par recombinaison avec le glycérol. Mises sous une forme appropriée à leur utilisation, les fractions enrichies en acides gras polyinsaturés peuvent constituer des produits nutritifs, cosmétiques, dermatologiques ou pharmaceutiques, décrits par exemple dans les brevets européens EP-A-92.085 et 92.076. Ces produits peuvent être administrés par voie orale, entérale, parentérale ou topique.

Les fractions d'acides gras peuvent également servir de produit de départ dans la synthèse d'acides gras rares, par exemple de l'acide dihomo-gamma-linolénique à partir d'une fraction enrichie en acide gamma-linolénique de manière connue, par voie synthétique ou enzymatique.

Les exemples ci-après illustrent l'invention. Dans ceux-ci, les pourcentages et parties sont en poids, sauf indication contraire.

Exemple 1

Dans une cuve à double manteau, on ajoute à 100 kg d'huile de pépins de cassis complètement raffinée (décolorée et purifiée) 213 kg d'une solution contenant 101,5 kg d'eau, 30,6 kg d'hydroxyde de sodium, 80,6 kg d'éthanol et 0,3 kg d'éthylènediaminetétraacétate disodique sous brassage lent et on chauffe la solution à 60°C pendant 30 mins. On refroidit ensuite la solution à 30°C avec 40 kg d'eau froide, puis on l'acidifie jusqu'à pH 1 par 100 kg d'une solution aqueuse d'acide chlorhydrique à 32% qu'on ajoute lentement. Après addition de 167 kg d'hexane, on agite fortement le mélange pendant 1 h à 30°C. On laisse les phases se séparer pendant 15 mins. et on élimine la phase aqueuse. Après évaporation de l'hexane de la phase organique sous le vide de la trompe à eau à 40°C, on recueille 90 kg d'acides gras.

On ajoute à 10 kg des acides gras précédents, 30 kg d'urée et 63 kg de méthanol et on chauffe à 65°C sous fort brassage pendant 20-30 mins. jusqu'à l'obtention d'une solution limpide.

On pompe alors la solution à travers un échangeur de chaleur à surface raclée du type KOMBINATOR ® dans les conditions suivantes:

| Température à la sortie: | 1°C |
|---|---|
| Vitesse de rotation des couteaux: | 500 t/min |
| Débit: | 35 kg/h |
| Fluide frigorigène, fréon à: | - 15°C |

On dirige la dispersion sortant de l'appareil vers une cuve à double manteau refroidie à 2°C où elle réside 10 à 20 mins. On filtre alors la dispersion avec un filtre à poches GAF ®.

On évapore le méthanol à 40°C sous le vide de la trompe à eau, puis on reprend la phase liquide par 8 kg d'hexane et on ajoute 6,7 kg d'une solution aqueuse d'acide chlorhydrique à 32% jusqu'à l'obtention d'un pH de 1 et 18 kg d'eau.

On laisse les phases se séparer, on recueille la phase organique et on ajoute 8 kg d'hexane à la phase aqueuse. Après agitation, on recueille la phase organique que l'on joint à la phase organique précédente puis on évapore l'hexane à 40°C sous le vide de la trompe à eau.

Les pourcentages d'acides gamma-linolénique, C 18: 3Δ 6, 9, 12 (GLA) dans l'huile de départ et dans la fraction enrichie (déterminés par chromatographie en phase gazeuse) sont indiqués ci-après:

| | GLA (%) |
|---|---|
| Huile de pépins de cassis | 17,4 |
| Fraction enrichie | 65 |

Exemples 2-7

On procède comme à l'exemple 1 sauf que l'on fait passer la solution contenant les acides gras, l'urée et le méthanol à travers une ligne comprenant en série: un premier échangeur de chaleur à surface raclée de type VOTATOR ®(échangeur 1), un second échangeur de chaleur à surface raclée de type VOTATOR ®(échangeur 2) et une conduite (échangeur 3) refroidis à l'eau glacée à 1°C.

4

Les données concernant la matière première, les conditions du traitement et la fraction enrichie sont indiquées dans le tableau 1 ci-après (les pourcentages de GLA sont déterminés par chromatographie en phase gazeuse):

**TABLEAU 1**

| Exemples Matière première (M.P.) | % GLA de la M.P. | Débit (kg/h) | Echangeur 1 | | | Echangeur 2 | | | Echangeur 3 | | % GLA de la fraction enrichie | Rendement (%) |
|---|---|---|---|---|---|---|---|---|---|---|---|---|
| | | | T.entrée (C°) | T.sortie (C°) | Vitesse de rotat. du racleur (t/min) | T.entrée (C°) | T.sortie (C°) | Vitesse de rotat. du racleur (t/min) | T.entrée (C°) | T.sortie (C°) | | |
| 2. Acides gras d'huile de pépins de cassis | 17,4 | 21,6 | 63 | 30,1 | 500 | 30,1 | 9,9 | 1000 | 9,9 | 5,7 | 74,8 | 12,5 |
| 3. Acides gras d'huile de pépins de cassis | 17,4 | 21 | 28 | 18,3 | 500 | 17,6 | 6,3 | 1000 | - | - | 71,9 | 13,3 |
| 4. Acides gras d'huile de graines de consoude | 26,9 | 20 | 31 | 16,5 | 500 | 16,5 | 5,5 | 1000 | - | - | 94,2 | 16,5 |
| 5. Acides gras d'huile de graines de bourrache | 24,7 | 40 | 65 | 19 | 700 | 19 | 9 | 1000 | 10 | 3,9 | 94 | 15 |
| 6. Acides gras d'huile de graines d'onagre | 8 | 43 | 65 | 24 | 700 | 23,9 | 12 | 1000 | 12 | 5,8 | 68,3 | 9 |
| 7. Acides gras d'huile de graines d'onagre (fraction enrichie de l'exemple 6*) | 68,3 | 37 | 65 | 18,8 | 700 | 18,8 | 8,7 | 1000 | 8,7 | 3,5 | 93,8 | 45 |

- non déterminé

* Dans ce cas, le mélange de départ est une fraction enrichie qui est traitée une seconde fois.

Exemple 8

On applique le procédé des exemples 2 à 7 au fractionnement des acides gras d'huile de poisson sauf que l'on utilise trois échangeurs de chaleur à surface raclée de type VOTATOR ® en série refroidis par l'eau glacée à 1°C.

L'huile de poisson ne contient pas d'acide gamma-linolénique mais d'autres acides gras à activité biologique comme les acides éicosapentaénoïque C20:5 et docosahexaénoïque C22:6.

Les conditions du traitement sont indiquées dans le tableau 2 ci-après:

## Tableau 2

| Débit | Echangeur 1 | | | Echangeur 2 | | |
|---|---|---|---|---|---|---|
| kg/h | T.entrée (C°) | T.sortie (C°) | Vitesse (t/min) | T.entrée (C°) | T.sortie (C°) | Vitesse (t/min) |
| 49 | 65 | 37,3 | 700 | 37,2 | 23,7 | 1000 |

## Suite du tableau 2:

| Echangeur 3 | | | Rendement |
|---|---|---|---|
| T.entrée (C°) | T.sortie (C°) | Vitesse (t/min) | (%) |
| 23,7 | 8,9 | 500 | 18,9 |

Les compositions des acides gras du mélange de départ et de la fraction enrichie sont indiquées dans le tableau 3 ci-après (déterminées par chromatographie en phase gazeuse):

## Tableau 3

### Composition des acides gras (%)
--------------------------------

| Matière première | C18:4 | C20:5 | C22:6 | autres |
|---|---|---|---|---|
| | 1,7 | 13,9 | 13,6 | 70,8 |
| Fraction enrichie | 8,8 | 40,4 | 37,3 | 13,5 |

Exemple 9

On procède comme à l'exemple 1 au fractionnement des acides gras de l'huile de lin en utilisant un échangeur de chaleur à surface raclée de type VOTATOR® refroidi à l'eau glacée à 1°C.

Les pourcentages d'acide alpha-linolénique (α-LA) de la matière première et de la fraction enrichie (déterminés par chromatographie en phase gazeuse), ainsi que les paramètres du procédé sont indiqués dans le tableau 4 ci-après:

## Tableau 4

| % de LA de la ma-tière pre-mière | Débit (kg/h) | Echangeur | | | % de LA de la frac-tion enri-chie | Rendement (%) |
|---|---|---|---|---|---|---|
| | | T.entrée (C°) | T.sortie (C°) | Vitesse (t/min) | | |
| 54,5 | 66 | 40 | 16,6 | 700 | 87,6 | 18,1 |

Exemple 10

On procède comme à l'exemple 1 au fractionnement d'un mélange d'acides gras provenant de lipides extraits du placenta de mammifères. Ce mélange contient un pourcentage élevé d'acide arachidonique C20:4 (AA) et d'acide dihomo-gamma-linolénique C20:3 (DHGLA). On utilise un échangeur de chaleur à surface raclée de type VOTATOR ® refroidi à l'éthanol à - 16°C.

Les conditions du traitement sont indiquées au tableau 5 ci-après:

## Tableau 5

| Débit (kg/h) | Echangeur | | | Rendement (%) |
|---|---|---|---|---|
| | T.entrée (C°) | T.Sortie (C°) | Vitesse (t/min) | |
| 28 | 40 | 4 | 900 | 7 |

Les pourcentages des principaux acides gras polyinsaturés de la matière première et de la fraction enrichie (déterminés par chromatographie en phase gazeuse) sont indiqués dans le tableau 6 ci-après:

## Tableau 6

| Acides gras | Matière première (%) | Fraction enrichie (%) |
|---|---|---|
| C18:2 | 12,2 | 17,8 |
| C20:3 (DHGLA) | 5,1 | 9,6 |
| C20:4 (AA) | 18 | 35,7 |
| C22:6 | 8,2 | 17,4 |
| autres | 56,5 | 19,5 |

Exemple 11

On procède comme à l'exemple 1 au fractionnement d'un mélange d'acides gras provenant de l'huile de pépins de quandong. Ce mélange a la particularité de contenir des acides acétyléniques, en particulier l'acide santalbique ou trans-11-octadécen-9-ynoïque.

On utilise un échangeur de chaleur à surface raclée de type VOTATOR ® refroidi à l'éthanol à - 16°C. Les conditions du traitement ainsi que les pourcentages d'acide santalbique de la matière première et de la fraction enrichie (déterminés par chromatographie en phase gazeuse) sont indiqués au tableau 7 ci-après:

8

## Tableau 7

| % d'acide santalbique de la matière première | Débit (kg/h) | Echangeur | | | % d'acide santalbique de la fraction enrichie | Rendement (%) |
|---|---|---|---|---|---|---|
| | | T.entrée (C°) | T.Sortie (C°) | Vitesse (t/min) | | |
| 38,8 | 40 | 45 | 10 | 800 | 87,6 | 9 |

Exemples 12-13

12.

On procède au fractionnement de l'huile de pépins de cassis comme à l'exemple 2 sauf que l'on sépare la phase liquide d'avec la phase solide par centrifugation à 4°C à 5000 t/mins pendant 10 min au lieu de filtrer. La fraction obtenue contient 74% de GLA (déterminé par chromatographie en phase gazeuse).

13.

On procède au fractionnement de l'huile de pépins de cassis comie à l'exemple 2 sauf que l'on récupère les acides gras contenus dans la phase solide. Pour ce faire, on chauffe 1 partie de celle-ci à 80°C avec 2 parties d'eau, on ajoute 0,6 partie d'hexane, on agite et on sépare la phase organique dont on évapore l'hexane à 40°C sous le vide de la trompe à eau. On ajoute alors les acides gras ainsi récupérés à la même quantité d'acides gras frais non traités.

Le fractionnement conduit à un mélange contenant 67% de GLA (déterminé par chromatographie en phase gazeuse) avec un rendement de 12,5%. On obtient ainsi la même quantité de GLA avec environ 25% de moins d'acides gras frais.

## Revendications

1. Procédé de fractionnement en continu d'un mélange d'acides gras ou des dérivés d'acides gras polyinsaturés, dans lequel on fait réagir le mélange avec l'urée en tant qu'agent complexant en solution dans un milieu réactionnel, dans les conditions permettant la formation dudit complexe, à 60-65°C, et l'on forme le dit complexe insoluble en refroidissant le milieu réactionnel, caractérisé par le fait que l'on refroidit le milieu réactionnel dans un à cinq échangeurs à surface râclée placés en série de sorte que la température de la charge a'la sortie du dervier échangeur est de -5 à +15°C, que la durée de résidence totale de la charge dans les échangeurs est de quelques secondes à quelques minutes, que l'on sépare le complexe d'inclusion formé et que l'on recueille une fraction enrichie en acides gras polyinsaturés dans la phase liquide.

2. Procédé selon la revendication1, caractérisé par le fait que l'on prépare une solution saturée d'urée dans le méthanol, que l'on ajoute le mélange d'acides gras ou de dérivés d'acides gras, que l'on pompe la solution portée à une température de 60-65°C à travers un à cinq échangeurs à surface râclée refroidis par circulation d'un fluide frigorigène de température -25 à +5°C, de sorte que la solution se transforme en dispersion sous l'effet du refroidissement et que la température de la dispersion à la sortie du dernier échangeur est -5 à +15°C.

3. Procédé selon la revendication 1 ou 2, caractérisé par le fait que l'on remplace l'échangeur à surface râclée en bout de ligne par une conduite d'attente munie de moyens de refroidissement.

**Claims**

1. A process for the continuous fractionation of a mixture of polyunsaturated fatty acids or derivatives thereof, in which the mixture is reacted with urea as complexing agent in solution in a reaction medium under conditions allowing the formation of said complex at a temperature in the range from 60 to 65°C and the said insoluble complex is formed by cooling of the reaction medium, characterized in that the reaction medium is cooled in one to five scraped-surface heat exchangers arranged in line in such a way that the temperature of the batch on leaving the last exchanger is -5 to +15°C, in that the total residence time of the batch in the exchangers is a few seconds to a few minutes, in that the inclusion complex formed is separated and in that a fraction enriched with polyunsaturated fatty acids is collected in the liquid phase.

2. A process as claimed in claim 1, characterized in that a saturated solution of urea in methanol is prepared, the mixture of fatty acids or fatty acid derivatives is added, in that the solution brought to a temperature of 60 to 65°C is pumped through one to five cooled scraped-surface heat exchangers brought by circulation of a refrigerating fluid to a temperature of -25 to +5°C so that the solution changes into a dispersion under the cooling effect and in that the temperature of the dispersion leaving the last exchanger is -5 to +15°C.

3. A process as claimed in claim 1 or 2, characterized in that the end-of-line heat exchanger is replaced by a holding pipe equipped with cooling means.

**Patentansprüche**

1. Verfahren zur kontinuierlichen Fraktionierung einer Mischung mehrfach ungesättigter Fettsäuren oder Fettsäurederivate, bei dem man die Mischung in Lösung mit Harnstoff als Komplexierungsmittel in einem Reaktionsmedium, in dem die Bedingungen die Bildung des Komplexes gestatten, bei 60-65°C umsetzt und man den genannten Komplex durch Abkühlen des Reaktionsmediums unlöslich macht, dadurch gekennzeichnet, daß man das Reaktionsmedium in von einem bis zu fünf Wärmeaustauschern mit glatter Oberfläche, die in Reihe angeordnet sind, so abkühlt, daß die Charge am Ausgang des letzten Wärmeaustauschers eine Temperatur von -5°C bis +15°C aufweist, daß die Gesamtverweilzeit der Charge in den Wärmeaustauschern zwischen einigen Sekunden und einigen Minuten liegt, daß man den gebildeten Einschlußkomplex abtrennt und daß man in der flüssigen Phase eine mit mehrfach ungesättigten Fettsäuren angereicherte Fraktion gewinnt.

2. Verfahren nach Anspruch 1, dadurch gekennzeichnet, daß man eine gesättigte Lösung von Harnstoff in Methanol herstellt, daß man die Mischung der Fettsäuren oder der Fettsäurederivate zusetzt, daß man die Trägerlösung bei einer Temperatur von 60-65°C so durch einen bis fünf Wärmeaustauscher mit glatter Oberfläche, die durch Umwälzung einer Kühlflüssigkeit mit einer Temperatur zwischen -25°C und +5°C gekühlt werden, pumpt, daß die Lösung infolge der Abkühlung in eine Dispersion überführt wird und daß die Temperatur der Dispersion am Ausgang des letzten Wärmeaustauschers zwischen -5°C und +15°C liegt.

3. Verfahren nach Anspruch 1 der 2, dadurch gekennzeichnet, daß man den am Ende der Reihe angeordneten Wärmeaustauscher mit glatter Oberfläche durch ein mit Mitteln zu seiner Kühlung versehenes Verweilrohr ersetzt.